(19) Europäisches Patentamt
European Patent Office
Office européen des brevets

(11) Veröffentlichungsnummer : **0 435 824 B1**

# (12) EUROPÄISCHE PATENTSCHRIFT

(45) Veröffentlichungstag der Patentschrift : **30.03.94 Patentblatt 94/13**

(51) Int. Cl.$^5$ : **C07C 331/28**

(21) Anmeldenummer : **90811000.0**

(22) Anmeldetag : **18.12.90**

## (54) Verfahren zur Herstellung von Isothiocyanaten.

(30) Priorität : **28.12.89 US 458124**

(43) Veröffentlichungstag der Anmeldung : **03.07.91 Patentblatt 91/27**

(45) Bekanntmachung des Hinweises auf die Patenterteilung : **30.03.94 Patentblatt 94/13**

(84) Benannte Vertragsstaaten : **AT BE CH DE DK ES FR GB GR IT LI LU NL SE**

(56) Entgegenhaltungen :
**EP-A- 0 210 487**
**DE-A- 1 618 037**
**FR-A- 2 288 739**
**CHEMISTRY AND INDUSTRY, Band 27, 3. Juli 1954, Seite 785, London, GB; J.N. BAXTER et al.: "A new method of preparation of aryl isothiocyanates"**

(73) Patentinhaber : **CIBA-GEIGY AG**
**Klybeckstrasse 141**
**CH-4002 Basel (CH)**

(72) Erfinder : **Hässig, Robert, Dr.**
**Gänsackerring 3**
**CH-5264 Gipf-Oberfrick (CH)**

EP 0 435 824 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft ein Verfahren zur Herstellung von Isothiocyanaten der Formel I

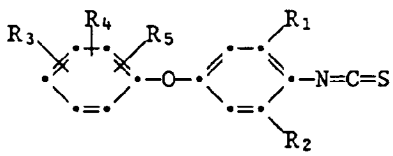

(I)

in welcher $R_1$ und $R_2$ unabhängig voneinander je $C_1$-$C_6$-Alkyl bedeuten und $R_3$, $R_4$ und $R_5$ unabhängig voneinander je Wasserstoff, Halogen, $C_1$-$C_4$-Alkyl, $C_1$-$C_4$-Alkoxy, Trifluormethyl oder Nitro darstellen.

Die Isothiocyanate der Formel I sind wertvolle Zwischenprodukte zur Herstellung von pestiziden Wirkstoffen. Beispielsweise können sie durch Umsetzung mit Mono- oder Dialkylaminen und gegebenenfalls weitere Umsetzung mit Alkylhalogeniden in die entsprechenden 1,3-disubstituierten Thioharnstoffe bzw. Isothioharnstoffe mit ausgeprägter insektizider und akarizider Wirkung übergeführt werden. Solche insektiziden und akariziden Wirkstoffe, ihre Herstellung und Verwendung sind beispielsweise in der deutschen Offenlegungsschrift 3034905 beschrieben.

Es ist bekannt, Isothiocyanate durch Umsetzung von primären Aminen mit Thiophosgen herzustellen (vgl. Houben-Weyl, Methoden der organischen Chemie, IX, 875 (1955)). Obwohl auf diese Weise Isothiocyanate in der Regel mit sehr guten Ausbeuten hergestellt werden können, ist die Methode für eine wirtschaftliche Herstellung von Isothiocyanaten in technischem Massstab wegen des hohen Preises des Thiophosgens und seiner schwierigen Handhabung nachteilig.

Es ist ferner bekannt, primäre Amine, insbesondere primäre aromatische Amine, in Form ihrer Salze in einem inerten Lösungsmittel mit Ammonium- oder Alkalithiocyanaten zu den entsprechenden asymmetrisch substituierten Thioharnstoffen umzusetzen (vgl. Houben-Weyl, Methoden der organischen Chemie, IX, 888 (1955)) und diese durch Abspaltung von Ammoniak in die entsprechenden Isothiocyanate überzuführen (vgl. Chemistry and Industry, 27, 785 (1954)). Mit der Kombination dieser bekannten Verfahrensschritte werden zwar die mit der Verwendung von Thiophosgen verbundenen Nachteile vermieden, die Bildung des Thioharnstoffs verläuft jedoch auch bei Verwendung von aromatischen Aminen mit unbefriedigenden Ausbeuten.

Es war daher die Aufgabe der vorliegenden Erfindung, ein Verfahren zur Herstellung von Isothiocyanaten der Formel I bereitzustellen, das von leicht zugänglichen, billigen und leicht handhabbaren Zwischenprodukten ausgeht und das die Herstellung der Isothiocyanate der Formel I in einfacher Weise und in guter Ausbeute ermöglicht.

Es wurde gefunden, dass die Umsetzung der in Isothiocyanaten der Formel I zugrundeliegenden Amine mit Ammonium- oder Alkalithiocyanaten praktisch quantitativ zu den entsprechenden Thioharnstoffen verläuft, wenn man diese Umsetzung in einem inerten Lösungsmittel in Gegenwart von 0,5-5 Gew.% Wasser bezogen auf das Gesamtgewicht des Reaktionsgemisches durchführt.

Das erfindungsgemässe Verfahren zur Herstellung von Isothiocyanaten der Formel I ist daher dadurch gekennzeichnet, dass man ein Amin der Formel II

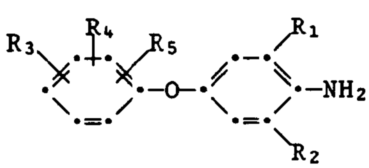

(II)

in welcher $R_1$, $R_2$, $R_3$, $R_4$ und $R_5$ die in der Formel I angegebene Bedeutung haben, in Gegenwart von mindestens der äquivalenten Menge einer Säure und in Gegenwart von 0,5-5 Gew.% Wasser bezogen auf das Gesamtgewicht des Reaktionsgemisches in einem inerten Lösungsmittel mit Ammonium- oder Alkalithiocyanat zu einem Thioharnstoff der Formel III

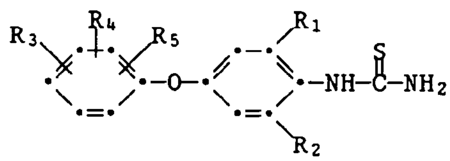

(III)

in welcher $R_1$, $R_2$, $R_3$, $R_4$ und $R_5$ die unter Formel I angegebene Bedeutung haben, umsetzt und diesen thermisch zu einem Isothiocyanat der Formel I und Ammoniak spaltet.

Unter den nach dem erfindungsgemässen Verfahren herstellbaren Isothiocyanaten der Formel I sind diejenigen bevorzugt, in welchen $R_1$ und $R_2$ je $C_2$-$C_4$-Alkyl, insbesondere Aethyl, n-Propyl, Isopropyl, n-Butyl, Isobutyl, sek.Butyl, tert.Butyl bedeuten, $R_3$ für Wasserstoff, Chlor, Methoxy, Trifluormethyl oder Nitro und $R_4$ und $R_5$ je für Wasserstoff oder Chlor stehen. Besonders bevorzugt sind Isothiocyanate der Formel I in welchen $R_1$ und $R_2$ je Isopropyl oder sek.Butyl bedeuten, und $R_3$ Wasserstoff, Chlor, Trifluormethyl, $R_4$ Wasserstoff oder Chlor und $R_5$ Wasserstoff darstellt. Ganz besonders bevorzugt ist die Verbindung 2,6-Diisopropyl-4-phenoxyphenylisothiocyanat.

Als Säuren, in deren Gegenwart die Umsetzung der Amine der Formel II mit Ammonium- oder einem Alkalithiocyanat durchgeführt werden kann, sind allgemein starke, nicht oxidierende Mineralsäuren geeignet. Bevorzugte Säuren sind Chlorwasserstoff, Bromwasserstoff und Schwefelsäure. Besonders bevorzugt ist Chlorwasserstoff. Die Säuren können in stöchiometrischer Menge oder im Ueberschuss eingesetzt werden. Vorteilhaft liegt das Molverhältnis von Amin der Formel II zu Säure zwischen 1:1-1:1,25. Bevorzugt beträgt das Molverhältnis von Amin der Formel II zu Säure 1:1,05-1:1,15.

Die Amine der Formel II werden vorzugsweise in Form ihrer Salze, insbesondere in Form ihrer Hydrochloride eingesetzt.

Vorzugsweise wird die Umsetzung der Amine der Formel II in einem inerten, mit Wasser nicht mischbaren organischen Lösungsmittel durchgeführt. Geeignete Lösungsmittel sind aromatische Kohlenwasserstoffe, insbesondere Alkylbenzole oder Gemische von Alkylbenzolen mit einem Siedepunkt von 110-170°C, wie Toluol, Aethylbenzol, Xylole, Cumol oder Trimethylbenzole oder Gemische solcher Alkylbenzole. Weitere geeignete Lösungsmittel sind aliphatische und aromatische Halogenkohlenwasserstoffe, wie Methylenchlorid, Chloroform, Kohlenstofftetrachlorid, 1,2-Dichlorethan, Chlorbenzol und o-Dichlorbenzol. Bevorzugte Lösungsmittel sind Toluol und Xylole, sowie Gemische von Alkylbenzolen. Besonders bevorzugte Lösungsmittel sind Xylole und Gemische von Alkylbenzolen mit einem Siedepunkt im Bereich von 110-170°C. Geeignete Gemische von Alkylbenzolen sind insbesondere die unter der Bezeichnung Solvesso® 100 (Esso) und Shellsol® A (Shell) erhältlichen Gemische mit einem Siedepunkt von 150-170°C.

Die Wassermenge, in deren Gegenwart die Umsetzung des Amins der Formel II mit dem Ammonium- oder Alkalithiocyanat durchgeführt wird, beträgt vorzugsweise 1-2 Gew.% bezogen auf das Gesamtgewicht des Reaktionsgemisches.

Als Alkalithiocyanate kommen insbesondere Natrium- und Kaliumthiocyanat in Betracht. Vorzugsweise verwendet man Natriumthiocyanat. Das Ammonium-oder Alkalithiocyanat kann in stöchiometrischer Menge eingesetzt werden. Vorzugsweise verwendet man jedoch pro Mol Amin der Formel II 1,05-1,25 Mol Ammonium- oder Alkalithiocyanat.

Die Umsetzung des Amins der Formel II mit dem Ammonium- oder Alkalithiocyanat wird vorteilhaft bei einer Temperatur von 80-120°C durchgeführt. Vorzugsweise führt man die Umsetzung des Amins der Formel II mit dem Ammonium- oder Alkalithiocyanat bei einer Temperatur von 90-110°C durch.

Die Spaltung des Thioharnstoffs der Formel III wird vorteilhaft bei einer Temperatur von 125-170°C, vorzugsweise bei 140-160°C in einem inerten Lösungsmittel vorgenommen. Geeignete inerte Lösungsmittel, in denen die Spaltung der Thioharnstoffe der Formel III durchgeführt werden kann, sind aromatische Kohlenwasserstoffe, insbesondere Alkylbenzole oder Gemische von Alkylbenzolen mit einem Siedepunkt im Bereich von 125-170°C, wie Aethylbenzol, Xylole, Trimethylbenzole, Aethyl-methylbenzole und Cumol, sowie Gemische solcher Alkylbenzole. Weitere geeignete Lösungsmittel für die thermische Spaltung der Harnstoffe der Formel III sind aromatische Halogenkohlenwasserstoffe, wie Chlorbenzol und o-Dichlorbenzol. Bevorzugte Lösungsmittel, in denen die thermische Spaltung der Thioharnstoffe der Formel III durchgeführt werden kann, sind Chlorbenzol, Xylole und Gemische von Alkylbenzolen, z.B. die im Handel unter der Bezeichnung Solvesso® 100 und Shellsol® A erhältlichen Gemische mit einem Siedepunkt von 150-170°C. Die Spaltungsreaktion nimmt je nach Reaktionstemperatur 3-6 Stunden in Anspruch.

Das Verfahren wird in der Regel bei Normaldruck durchgeführt. Die Spaltung des Thioharnstoffs der Formel III wird jedoch vorteilhaft bei leicht vermindertem Druck durchgeführt. Vorzugsweise wird die Spaltung des Thioharnstoffs der Formel III bei einem Druck von 0,8-1 bar durchgeführt.

Der Thioharnstoff der Formel III kann durch Filtration oder Abdampfen des Lösungsmittels isoliert werden. Das Produkt wird nach Entfernung des gebildeten Alkali- oder Ammoniumsalzes durch Waschen mit Wasser in guter Reinheit und in Ausbeuten von 95-97 % der Theorie erhalten. Es ist jedoch auch möglich, den Thioharnstoff der Formel III unmittelbar im Anschluss an seine Bildung nach Abtrennung des im Reaktionsgemisch vorhandenen Wassers ohne Isolierung direkt zum Isothiocyanat der Formel I zu spalten. Das Isothiocyanat der Formel I kann in einfacher Weise durch Abdestillieren des Lösungsmittels und Destillation des Rückstandes im Vakuum gewonnen werden.

Nach einer bevorzugten Variante des erfindungsgemässen Verfahrens werden Verbindungen der Formel Ia

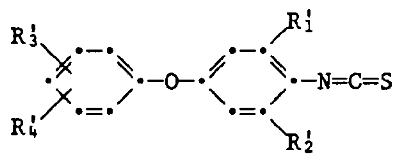

(Ia)

in welcher $R_1'$ und $R_2'$ je Isopropyl und sek.Butyl, $R_3'$ Wasserstoff, Chlor oder Trifluormethyl und $R_4'$ Wasserstoff oder Chlor bedeuten, hergestellt, indem man ein Amin der Formel IIa

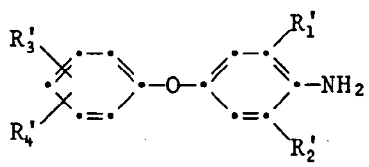

(IIa)

in welcher $R_1'$, $R_2'$, $R_3'$ und $R_4'$ die unter Formel Ia angegebene Bedeutung haben, bei einer Temperatur von 90-110°C in Toluol, Xylol oder einem Gemisch von Alkylbenzolen als Lösungsmittel in Gegenwart von 1,05-1,15 Mol Chlorwasserstoff pro Mol Amin der Formel IIa mit 1,05-1,25 Mol Natriumthiocyanat pro Mol Amin der Formel IIa zum Thioharnstoff der Formel IIIa

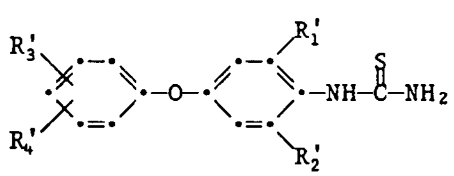

(IIIa)

worin $R_1'$, $R_2'$, $R_3'$ und $R_4'$ die unter Formel Ia angegebene Bedeutung haben, umsetzt und diesen in Xylol oder einem Gemisch von Alkylbenzolen bei 140-160°C thermisch zum Isothiocyanat der Formel Ia spaltet.

Durch das erfindungsgemässe Verfahren wird es möglich, die Isothiocyanate der Formel I ausgehend von Aminen der Formel II unter Vermeidung der Nachteile der bekannten Verfahren in einfacher Weise und in ausgezeichneter Ausbeute herzustellen.

Das erfindungsgemässe Verfahren wird durch die nachfolgenden Beispiele näher erläutert.

Beispiel 1: Herstellung von N-(4-Phenoxy-2,6-diisopropylphenyl)-thioharnstoff

305 g (1 Mol) 4-Phenoxy-2,6-diisopropylanilin-Hydrochlorid, werden zusammen mit 97 g (1,2 Mol) Natriumthiocyanat, 10 ml Wasser und 10 ml konzentrierter Salzsäure in 800 ml o-Xylol suspendiert. Die Mischung wird 6 Stunden auf 90°C erwärmt, wobei nach 3 Stunden und danach nach jeder weiteren Stunde 2 ml konzentrierte Salzsäure zugesetzt werden. Anschliessend wird das Reaktionsgemisch auf Raumtemperatur gekühlt, das Produkt abfiltriert, mit Wasser gewaschen und getrocknet. Man erhält 315 g (96 % der Theorie) N-(4-Phenoxy-2,6-diisopropylphenyl)-thioharnstoff vom Schmelzpunkt 219-221°C in Form von hellgrauen Kristallen.

Das als Ausgangsmaterial verwendete 4-Phenoxy-2,6-diisopropylanilin-Hydrochlorid kann wie folgt hergestellt werden:

Einer Lösung von 141 g (1,5 Mol) Phenol in 500 g Toluol werden unter Rühren zunächst 21 g (0,15 Mol) Kaliumcarbonat und dann 168 g (1,5 Mol) 50%ige wässrige Kaliumhydroxidlösung zugesetzt. Anschliessend wird unter Rückfluss das gesamte Wasser ausgekreist und dann etwa 400 g Toluol abdestilliert. Der Rückstand wird in 700 g Dimethylformamid gelöst. Nach Zugabe von 6 g (0,05 Mol) Kupfercarbonat wird solange Lösungsmittel abdestilliert, bis die Innentemperatur 140°C erreicht hat. Dann gibt man bei 140°C 256 g (1 Mol) 4-Brom-2,6-diisopropylanilin zu und hält die Mischung 10 h bei 140°C. Danach wird das Dimethylformamid im Vakuum abdestilliert, der Rückstand mit Wasser versetzt und das Produkt in Toluol aufgenommen. Das nach Abdampfen des Toluols erhaltene Rohprodukt wird durch Destillation im Vakuum gereinigt. Man erhält 215 g (80 % der Theorie) 4-Phenoxy-2,6-diisopropylanilin vom Siedepunkt 142-145°C/0,04 mbar und vom Schmelzpunkt 69-71°C als hellrotes Produkt, das durch Auflösen in 300 g Butylacetat, Zugabe von 115 g 37%iger Salzsäure und Auskreisen des Wassers durch Vakuumdestillation in das Hydrochlorid übergeführt wird. Dieses wird abfiltriert, mit Butylacetat gewaschen und im Vakuum getrocknet. Man erhält 235 g (96 % der Theorie bezogen auf eingesetztes 4-Phenoxy-2,6-diisopropylanilin) 4-Phenoxy-2,6-diisopropylanilin-Hydrochlorid vom Schmelzpunkt 247-249°C in Form von weissen Kristallen.

Beispiel 2: Herstellung von 4-Phenoxy-2,6-diisopropylphenylisothiocyanat

315 g (1 Mol) N-(4-Phenoxy-2,6-diisopropylphenyl)-thioharnstoff werden in 800 ml o-Xylol suspendiert und 5 Stunden auf Rückflusstemperatur erhitzt, wobei die Innentemperatur auf etwa 150°C ansteigt. Anschliessend wird das Reaktionsgemisch abgekühlt, das Lösungsmittel im Vakuum abdestilliert und das erhaltene Rohprodukt durch Destillation im Hochvakuum gereinigt. Man erhält 278 g (93 % der Theorie) 4-Phenoxy-2,6-diisopropylphenylisothiocyanat vom Siedepunkt 130-140°C/0,05 mbar als hellgelbes Oel.

Beispiel 3: Herstellung von 4-Phenoxy-2,6-diisopropylphenylisothiocyanat

315 g (1 Mol) N-(4-phenoxy-2,6-diisopropylphenyl)-thioharnstoff werden in 800 mml Solvesso® 100 (Gemisch von Alkylbenzolen mit einem Siedepunkt von 150-170°C) suspendiert. Danach wird die Mischung bei einem Druck von 800 mbar 3 Stunden auf 155-160°C erwärmt. Anschliessend wird das Lösungsmittel bei einem Druck von 200 mbar abdestilliert und der Rückstand durch Destillation im Hochvacuum gereinigt. Man erhält 280 g (94 % der Theorie) 4-Phenoxy-2,6-diisopropylphenylisothiocyanat als hellgelbes Oel vom Siedepunkt 130-140°C/0,05 mbar.

## Patentansprüche

1. Verfahren zur Herstellung von Isothiocyanaten der Formel I

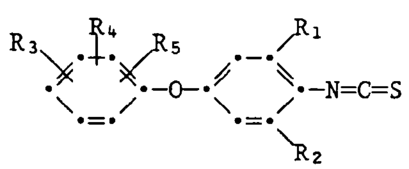

(I)

in welcher $R_1$ und $R_2$ unabhängig voneinander je $C_1$-$C_6$-Alkyl bedeuten und $R_3$, $R_4$ und $R_5$ unabhängig voneinander je Wasserstoff, Halogen, $C_1$-$C_4$-Alkyl, $C_1$-$C_4$-Alkoxy, Trifluormethyl oder Nitro darstellen, dadurch gekennzeichnet, dass man ein Amin der Formel II

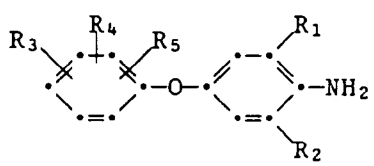

(II)

in welcher $R_1$, $R_2$, $R_3$, $R_4$ und $R_5$ die unter Formel I angegebene Bedeutung haben, in Gegenwart von mindestens der äquivalenten Menge einer Säure und in Gegenwart von 0,5-5 Gew.% Wasser bezogen auf das Gesamtgewicht des Reaktionsgemisches in einem inerten Lösungsmittel mit Ammonium- oder Alkalithiocyanat zu einem Thioharnstoff der Formel III

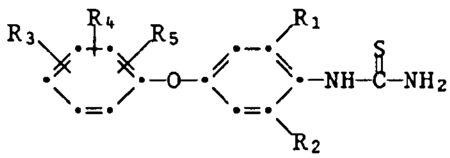

(III)

in welcher $R_1$, $R_2$, $R_3$, $R_4$ und $R_5$ die unter Formel I angegebene Bedeutung haben, umsetzt und diesen thermisch zu einem Isothiocyanat der Formel I und Ammoniak spaltet.

2. Verfahren nach Anspruch 1, dadurch gekennzeichnet, dass $R_1$ und $R_2$ je $C_2$-$C_4$-Alkyl, $R_3$ für Wasserstoff, Chlor, Methoxy, Trifluormethyl oder Nitro und $R_4$ und $R_5$ je für Wasserstoff oder Chlor stehen.

3. Verfahren nach Anspruch 1, dadurch gekennzeichnet, dass $R_1$ und $R_2$ je Isopropyl oder sek.Butyl, $R_3$ Wasserstoff, Chlor oder Trifluormethyl, $R_4$ Wasserstoff oder Chlor und $R_5$ Wasserstoff bedeutet.

4. Verfahren nach Anspruch 1, dadurch gekennzeichnet, dass $R_1$ und $R_2$ Isopropyl und $R_3$, $R_4$ und $R_5$ Wasserstoff bedeuten.

5. Verfahren nach Anspruch 1, dadurch gekennzeichnet, dass man die Umsetzung des Amins der Formel II mit Ammonium oder einem Alkalithiocyanat in Gegenwart von 1-1,25 Aequivalenten einer starken, nicht oxidierenden Mineralsäure pro Mol Amin der Formel II durchführt.

6. Verfahren nach Anspruch 5, dadurch gekennzeichnet, dass man pro Mol Amin der Formel II 1-1,25 äquivalente Chlorwasserstoff, Bromwasserstoff oder Schwefelsäure verwendet.

7. Verfahren nach Anspruch 5, dadurch gekennzeichnet, dass man pro Mol Amin der Formel II 1,05-1,15 Mol Chlorwasserstoff verwendet.

8. Verfahren nach Anspruch 1, dadurch gekennzeichnet, dass man die Amine der Formel II in Form ihrer Hydrochloride einsetzt.

9. Verfahren nach Anspruch 1, dadurch gekennzeichnet, dass man die Umsetzung eines Amins der Formel II mit Ammonium- oder einem Alkalithiocyanat in einem aromatischen Kohlenwasserstoff oder einem aliphatischen oder aromatischen Halogenkohlenwasserstoff als Lösungsmittel durchführt.

10. Verfahren nach Anspruch 9, dadurch gekennzeichnet, dass man die Umsetzung eines Amins der Formel II mit Ammonium- oder einem Alkalithiocyanat in Toluol, Xylol oder einem Gemisch von Alkylbenzolen als Lösungsmittel durchführt.

11. Verfahren nach Anspruch 9, dadurch gekennzeichnet, dass man die Umsetzung eines Amins der Formel II mit Ammonium- oder einem Alkalithiocyanat in Xylol oder einem Gemisch von Alkylbenzolen mit einem Siedepunkt von 150-170°C als Lösungsmittel durchführt.

12. Verfahren nach Anspruch 1, dadurch gekennzeichnet, dass man die Umsetzung des Amins der Formel II mit dem Ammonium- oder Alkalithiocyanat in Gegenwart von 1-2 Gew.% Wasser bezogen auf das Gesamtgewicht des Reaktionsgemisches durchführt.

13. Verfahren nach Anspruch 1, dadurch gekennzeichnet, dass man das Amin der Formel II mit Natriumthiocyanat umsetzt.

14. Verfahren nach Anspruch 1, dadurch gekennzeichnet, dass man pro Mol Amin der Formel II 1,05-1,25 Mol Ammonium- oder Alkalithiocyanat verwendet.

15. Verfahren nach Anspruch 1, dadurch gekennzeichnet, dass man das Amin der Formel II mit dem Ammonium- oder Alkalithiocyanat bei einer Temperatur von 80-120°C durchführt.

16. Verfahren nach Anspruch 15, dadurch gekennzeichnet, dass man die Umsetzung des Amins der Formel

II mit dem Ammonium- oder Alkalithiocyanat bei einer Temperatur von 90-110°C durchführt.

**17.** Verfahren nach Anspruch 1, dadurch gekennzeichnet, dass man die thermische Spaltung des Thioharnstoffs der Formel III bei einer Temperatur von 125-170°C durchführt.

**18.** Verfahren nach Anspruch 17, dadurch gekennzeichnet, dass man die Spaltung des Thioharnstoffs der Formel III bei einer Temperatur von 140-160°C durchführt.

**19.** Verfahren nach Anspruch 1, dadurch gekennzeichnet, dass man die Spaltung des Thioharnstoffs der Formel III bei einem Druck von 0,8-1 bar durchführt.

**20.** Verfahren nach Anspruch 1 zur Herstellung von Verbindungen der Formel Ia

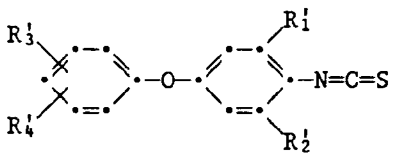

(Ia)

in welcher $R_1'$, $R_2'$ je Isopropyl und sek.Butyl, $R_3'$ Wasserstoff, Chlor oder Trifluormethyl und $R_4'$ Wasserstoff oder Chlor bedeuten, dadurch gekennzeichnet, dass man ein Amin der Formel IIa

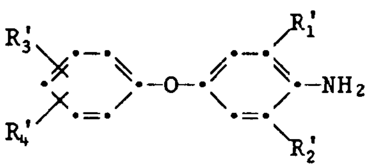

(IIa)

in welcher $R_1'$, $R_2'$, $R_3'$ und $R_4'$ die unter Formel Ia angegebene Bedeutung haben, bei einer Temperatur von 80-90°C in Toluol, Xylol oder einem Gemisch von Alkylbenzolen als Lösungsmittel in Gegenwart von 1,05-1,15 Mol Chlorwasserstoff pro Mol Amin der Formel IIa mit 1,05-1,25 Mol Natriumthiocyanat pro Mol Amin der Formel IIa zum Thioharnstoff der Formel IIIa

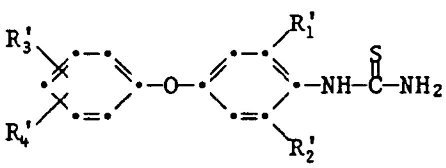

(IIIa)

worin $R_1'$, $R_2'$, $R_3'$ und $R_4'$ die unter Formel Ia angegebene Bedeutung haben, umsetzt und diesen in Xylol bei einer Temperatur von 140-150°C und einem Druck von 0,8-1 bar thermisch zum Isothiocyanat der Formel Ia spaltet.

## Claims

**1.** A process for the preparation of an isothiocyanate of formula I

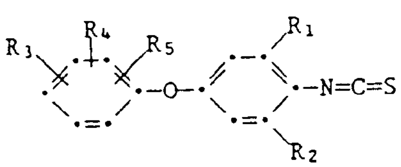

(I)

wherein $R_1$ and $R_2$ are each independently of the other $C_1$-$C_6$alkyl and $R_3$, $R_4$ and $R_5$ are each independently of the other hydrogen, halogen, $C_1$-$C_4$alkyl, $C_1$-$C_4$alkoxy, trifluoromethyl or nitro, which process comprises reacting an amine of formula II

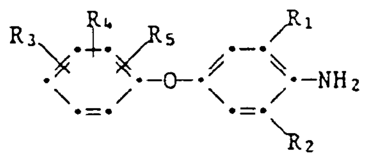

(II)

wherein $R_1$, $R_2$, $R_3$, $R_4$ and $R_5$ are as defined for formula I, in the presence of at least the equivalent amount of an acid and in the presence of 0.5-5% by weight of water, based on the total weight of the reaction mixture, in an inert solvent, with ammonium thiocyanate or an alkali metal thiocyanate to form a thiourea of formula III

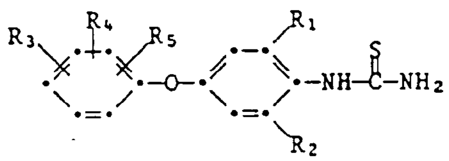

(III)

wherein $R_1$, $R_2$, $R_3$, $R_4$ and $R_5$ are as defined for formula I, and cleaving said thiourea by heating to give an isothiocyanate of formula I and ammonia.

2. A process according to claim 1, wherein $R_1$ and $R_2$ are each $C_2$-$C_4$alkyl, $R_3$ is hydrogen, chloro, methoxy, trifluoromethyl or nitro, and $R_4$ and $R_5$ are each hydrogen or chloro.

3. A process according to claim 1, wherein $R_1$ and $R_2$ are each isopropyl or sec-butyl, $R_3$ is hydrogen, chloro or trifluoromethyl, $R_4$ is hydrogen or chloro and $R_5$ is hydrogen.

4. A process according to claim 1, wherein $R_1$ and $R_2$ are isopropyl and $R_3$, $R_4$ and $R_5$ are hydrogen.

5. A process according to claim 1, wherein the reaction of the amine of formula II with ammonium thiocyanate or an alkali metal thiocyanate is carried out in the presence of 1-1.25 equivalents of a strong non-oxidising mineral acid per mol of amine of formula II.

6. A process according to claim 5, wherein 1-1.25 equivalents of hydrochloric acid, hydrobromic acid or sulfuric acid are used per mol of amine of formula II.

7. A process according to claim 5, wherein 1.05-1.15 mol of hydrochloric acid are used per mol of amine of formula II.

8. A process according to claim 1, wherein the amine of formula II is used in the form of its hydrochloride.

9. A process according to claim 1, wherein the reaction of an amine of formula II with ammonium thiocyanate or an alkali metal thiocyanate is carried out in an aromatic hydrocarbon or an aliphatic or aromatic halogenated hydrocarbon as solvent.

10. A process according to claim 9, wherein the reaction of an amine of formula II with ammonium thiocyanate or an alkali metal thiocyanate is carried out in toluene, xylene or a mixture of alkylbenzenes as solvent.

11. A process according to claim 9, wherein the reaction of an amine of formula II with ammonium thiocyanate or an alkali metal thiocyanate is carried out in xylene or a mixture of alkylbenzenes having a boiling range of from 150 to 170°C, as solvent.

12. A process according to claim 1, wherein the reaction of the amine of formula II with the ammonium thiocyanate or alkali metal thiocyanate is carried out in the presence of 1-2% by weight of water, based on

the total weight of the reaction mixture.

**13.** A process according to claim 1, wherein the amine of formula II is reacted with sodium thiocyanate.

**14.** A process according to claim 1, wherein 1.05-1.25 mol of ammonium thiocyanate or alkali metal thiocyanate are used per mol of amine of formula II.

**15.** A process according to claim 1, wherein the reaction of the amine of formula II with the ammonium thiocyanate or alkali metal thiocyanate is carried out in the temperature range from 80 to 120°C.

**16.** A process according to claim 15, wherein the reaction of the amine of formula II with the ammonium thiocyanate or alkali metal thiocyanate is carried out in the temperature range from 90 to 110°C.

**17.** A process according to claim 1, wherein the thermal cleavage of the thiourea of formula III is carried out in the temperature range from 125 to 170°C.

**18.** A process according to claim 17, wherein the cleavage of the thiourea of formula III is carried out in the temperature range from 140 to 160°C.

**19.** A process according to claim 1, wherein the cleavage of the thiourea of formula III is carried out under a pressure of 0.8-1 bar.

**20.** A process according to claim 1 for the preparation of a compound of formula Ia

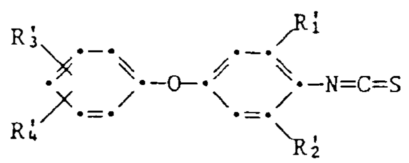

(Ia)

wherein $R'_1$ and $R'_2$ are each isopropyl or sec-butyl, R'3 is hydrogen, chloro or trifluoromethyl and $R'_4$ is hydrogen or chloro, which comprises reacting an amine of formula IIa

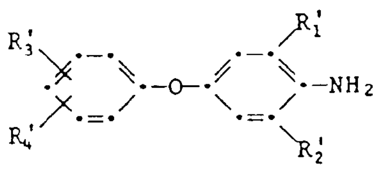

(IIa)

wherein $R'_1$, $R'_2$, $R'_3$ and $R'_4$ are as defined for formula Ia, in the temperature range from 80 to 90°C, in toluene, xylene or a mixture of alkylbenzenes as solvent, in the presence of 1.05 to 1.15 mol of hydrogen chloride per mol of amine of formula IIa, with 1.05 to 1.25 mol of sodium thiocyanate per mol of amine of formula IIa, to form the thiourea of formula IIIa

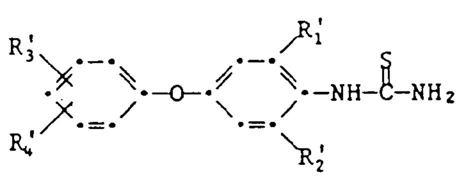

(IIIa)

wherein $R'_1$, $R'_2$, $R'_3$ and $R'_4$ are as defined for formula Ia, and cleaving said thiourea by heating in the temperature range from 140-150°C and under a pressure of 0.8-1 bar in xylene to form the isothiocyanate of formula Ia.

## Revendications

1. Procédé pour la préparation des isothiocyanates de formule I

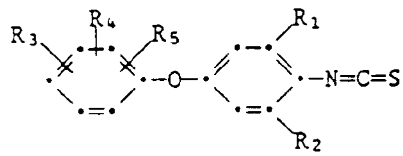

(I)

dans laquelle $R_1$ et $R_2$ représentent, indépendamment l'un de l'autre, un alkyle en $C_1$-$C_6$ et $R_3$, $R_4$ et $R_5$ représentent, indépendamment les uns des autres, l'hydrogène, un halogène, un alkyle en $C_1$-$C_4$, un alcoxy en $C_1$-$C_4$, le trifluorométhyle ou un nitro, caractérisé en ce que l'on fait réagir une amine de formule II

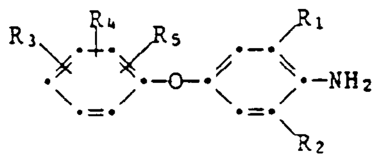

(II)

dans laquelle $R_1$, $R_2$, $R_3$, $R_4$ et $R_5$ ont les significations données pour la formule I, en présence d'au moins la quantité équivalente d'un acide et en présence de 0,5 à 5% en poids d'eau, rapporté au poids total du mélange réactionnel dans un solvant inerte avec le thiocyanate d'ammonium ou les thiocyanates alcalins pour former une thiourée de formule III

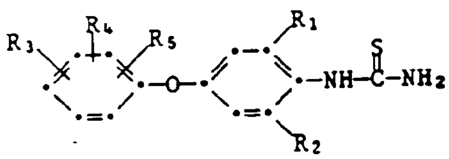

(III)

dans laquelle $R_1$, $R_2$, $R_3$, $R_4$ et $R_5$ ont les significations données pour la formule I et en ce que l'on clive thermiquement celle-ci pour former un isothiocyanate de formule I et de l'ammoniac.

2. Procédé selon la revendication 1, caractérisé en ce que $R_1$ et $R_2$ représentent un alkyle en $C_2$-$C_4$, $R_3$ représente l'hydrogène, le chlore, le méthoxy, le trifluorométhyle ou le nitro et $R_4$ et $R_5$ représentent l'hydrogène ou le chlore.

3. Procédé selon la revendication 1, caractérisé en ce que $R_1$ et $R_2$ représentent l'isopropyle ou le sec-butyle, $R_3$ représente l'hydrogène, le chlore ou le trifluorométhyle, $R_4$ représente l'hydrogène ou le chlore et $R_5$ représente l'hydrogène.

4. Procédé selon la revendication 1, caractérisé en ce que $R_1$ et $R_2$ représentent l'isopropyle et $R_3$, $R_4$ et $R_5$ représentent l'hydrogène.

5. Procédé selon la revendication 1, caractérisé en ce que l'on fait réagir l'amine de formule II avec le thiocyanate d'ammonium ou avec un thiocyanate alcalin en présence de 1 à 1,25 équivalent d'un acide minéral fort, non oxydant par mole d'amine de formule II.

6. Procédé selon la revendication 5, caractérisé en ce que l'on utilise par mole d'amine de formule II 1 à 1,25 équivalent d'acide chlorhydrique, d'acide bromhydrique ou d'acide sulfurique.

7. Procédé selon la revendication 5, caractérisé en ce que l'on utilise par mole d'amine de formule II 1,05 à 1,15 mole d'acide chlorhydrique.

8. Procédé selon la revendication 1, caractérisé en ce que l'on utilise les amines de formule II sous forme de leurs chlorhydrates.

9. Procédé selon la revendication 1, caractérisé en ce que l'on fait réagir une amine de formule II avec le thiocyanate d'ammonium ou avec un thiocyanate alcalin dans un hydrocarbure aromatique ou dans un hydrocarbure halogéné aliphatique ou aromatique comme solvant.

10. Procédé selon la revendication 9, caractérisé en ce que l'on fait réagir une amide de formule II avec le thiocyanate d'ammonium ou un thiocyanate alcalin dans le toluène, le xylène ou un mélange d'alkylbenzènes comme solvant.

11. Procédé selon la revendication 9, caractérisé en ce que l'on fait réagir une amine de formule II avec le thiocyanate d'ammonium ou avec un thiocyanate alcalin dans le xylène ou dans un mélange d'alkylbenzènes ayant un point d'ébullition compris entre 150 et 170°C comme solvant.

12. Procédé selon la revendication 1, caractérisé en ce que l'on fait réagir l'amine de formule II avec le thiocyanate d'ammonium ou un thiocyanate alcalin en présence de 1 à 2% en poids d'eau, rapporté au poids total du mélange réactionnel.

13. Procédé selon la revendication 1, caractérisé en ce que l'on fait réagir l'amine de formule II avec le thiocyanate de sodium.

14. Procédé selon la revendication 1, caractérisé en ce que l'on utilise par mole d'amine de formule II 1,05 à 1,25 mole de thiocyanate d'ammonium ou d'un thiocyanate alcalin.

15. Procédé selon la revendication 1, caractérisé en ce que l'on fait réagir l'amine de formule II avec le thiocyanate d'ammonium ou un thiocyanate alcalin à une température comprise entre 80 et 120°C.

16. Procédé selon la revendication 15, caractérisé en ce que l'on fait réagir l'amine de formule II avec le thiocyanate d'ammonium ou avec un thiocyanate alcalin à une température comprise entre 90 et 110°C.

17. Procédé selon la revendication 1, caractérisé en ce que l'on effectue le clivage thermique de la thiourée de formule III à une température comprise entre 125 et 170°C.

18. Procédé selon la revendication 17, caractérisé en ce que l'on effectue le clivage de la thiourée de formule III à une température comprise entre 140 et 160°C.

19. Procédé selon la revendication 1, caractérisé en ce que l'on effectue le clivage de la thiourée de formule III sous une pression comprise entre 0,8 et 1 bar.

20. Procédé selon la revendication 1 pour la préparation des composés de formule Ia

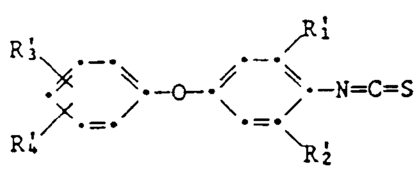

(Ia)

dans laquelle R'$_1$ et R'$_2$ représentent l'isopropyle et le sec-butyle, R'$_3$ représente l'hydrogène, le chlore ou le trifluorométhyle et R'$_4$ représente l'hydrogène ou le chlore, caractérisé en ce que l'on fait réagir une amine de formule IIa

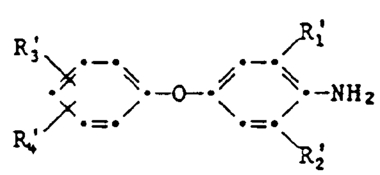

(IIa)

dans laquelle R'$_1$, R'$_2$, R'$_3$ et R'$_4$ ont les significations données pour la formule Ia, à une température comprise entre 80 et 90°C dans le toluène, le xylène ou un mélange d'alkylbenzènes, comme solvant, en présence de 1,05 à 1,15 mole d'acide chlorhydrique par mole d'amine de formule IIa avec 1,05 à 1,25 mole de thiocyanate de sodium par mole d'amine de formule IIa pour former une thiourée de formule IIIa

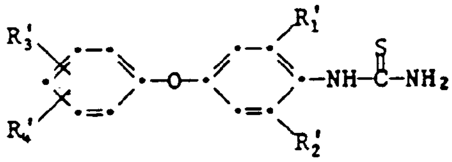

(IIIa)

où R'$_1$, R'$_2$, R'$_3$ et R'$_4$ ont les significations données pour la formule Ia et en ce que l'on clive thermiquement celle-ci dans le xylène à une température comprise entre 140 et 150°C et sous une pression comprise entre 0,8 et 1 bar pour former l'isothiocyanate de formule Ia.